# NOUVEAU FASCICULE DE BREVET EUROPEEN

(11) **EP 0 589 751 B2**
(45) Date de publication et mention de la décision concernant l'opposition: **31.01.2001**
(45) Mention de la délivrance du brevet: 19.07.1995
(21) Numéro de dépôt: 93402215.3
(22) Date de dépôt: 13.09.1993
(51) Int. Cl.: A61M 16/12

(54) **Installation et procédé de fourniture d'un mélange gazeux aux voies respiratoires d'un utilisateur**
Einrichtung und Verfahren zur Verabreichung eines Gasgemisches in die Atemwege eines Anwenders
Installation and procedure to deliver a gas mixture into the airways of a customer

(30) Priorité: 24.09.1992 FR 9211370
(43) Date de publication de la demande: 30.03.1994
(73) Titulaire: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: Briend, Robert, F-78340 Les-Clayes-sous-Bois (FR); Renaudin, Marie-Hélène, F-75015 Paris (FR)
(74) Mandataire: Le Moenner, Gabriel

(56) Documents cités:
- EP-A- 0 073 219
- EP-A- 0 183 593
- EP-A- 0 183 593
- EP-A- 0 347 282
- EP-A- 0 570 612
- WO-A-86/00537
- WO-A-92/10228
- WO-A-92/10228
- DE-C- 1 912 572
- US-A- 4 587 967
- Brochure "Die innovative Lösung für fortschrittliche Respirator-Therapie Servo Ventillator 300", Siemens-Elma AB, 1991, 4th edition, October 1992
- Brochure "Servo Ventilator 900/900B", mode d'emploi, pages 5, 16, 17, Siemens-Elema AB, AG1080, 4 octobre 1980
- Article: Frostell et al, "Inhaled nitric oxide dilates hypoxic pulmonary vasosonstriction without causing systemic vasodilation", Anesthesiology, V75, No. 3a, September 1991
- Article: Falke et al, "Inhaled nitric oxide selectively reduces pulmonary hypertension in severe ARDS and improves gas exchange as well as right heart ejection fraction - A case report", Am. rev. respir. dis., 1991: 143(4), suppl: A 248

## Description

La présente invention concerne une installation de fourniture d'un mélange gazeux aux voies respiratoires d'un utilisateur, comprenant un, dispositif, ou respirateur, de fourniture séquentielle d'un mélange respiratoire dans un embout d'utilisation, le dispositif comportant des moyens de détection de phases inspiratoires de l'utilisateur.

Une installation de ce type, pour la fourniture sous pression contrôlée d'air enrichi en oxygène ou d'oxygène aux voies respiratoires d'un utilisateur est décrite dans le document EP-A-347.282. Les installations actuelles de ce type sont avantageusement complétées par une sortie annexe de mélange respiratoire sous pression pour couplage à un circuit de nébulisation introduisant des gouttelettes de produit actif dans le mélange respiratoire lors des phases inspiratoires.

On a récemment constaté que le monoxyde d'azote, administré par inhalation, présente des propriétés vaso-dilatatrices pulmonaires pouvant se révéler fort utiles pour ia prise en charge de syndromes de détresse respiratoire de l'adulte et du nouveau-né ou pour le maintien des fonctions vitales lors des interventions chirurgicales sur l'ensemble coeur-poumon. Toutefois, en présence d'oxygène, le monoxyde d'azote s'oxyde rapidement pour forme du dioxyde d'azote, lequel peut provoquer la formation de métabolites toxiques.

La présente invention a pour objet de proposer une installation perfectionnée du type ci-dessus permettant de fournir, de façon simple et fiable et en toute sécurité, des quantités contrôlées de monoxyde d'azote sensiblement directement dans les voies respiratoires du patient, sans pré-mélange préalable et significatif avec l'oxygène du mélange respiratoire habituel, et lors des phases inspiratoires uniquement.

Pour ce faire, l'installation selon l'invention est telle que définie en revendication 1.

Selon d'autres caractéristiques de l'invention :
- les moyens d'adjonction du monoxyde d'azote comprennent une ligne de distribution entre la source de monoxyde d'azote et l'embout d'utilisation, cette ligne comportant un dispositif de régulation sensible à la délivrance d'un flux de mélange respiratoire par le dispositif de fourniture de mélange respiratoire ;
- le dispositif de régulation de fourniture de monoxyde d'azote comporte des moyens de détection du passage d'un flux dans une ligne de sortie annexe de mélange respiratoire du dispositif de fourniture.

La présente invention a pour autre objet un procédé de mise en oeuvre de l'installation, tel que defini dans la revendication 9.

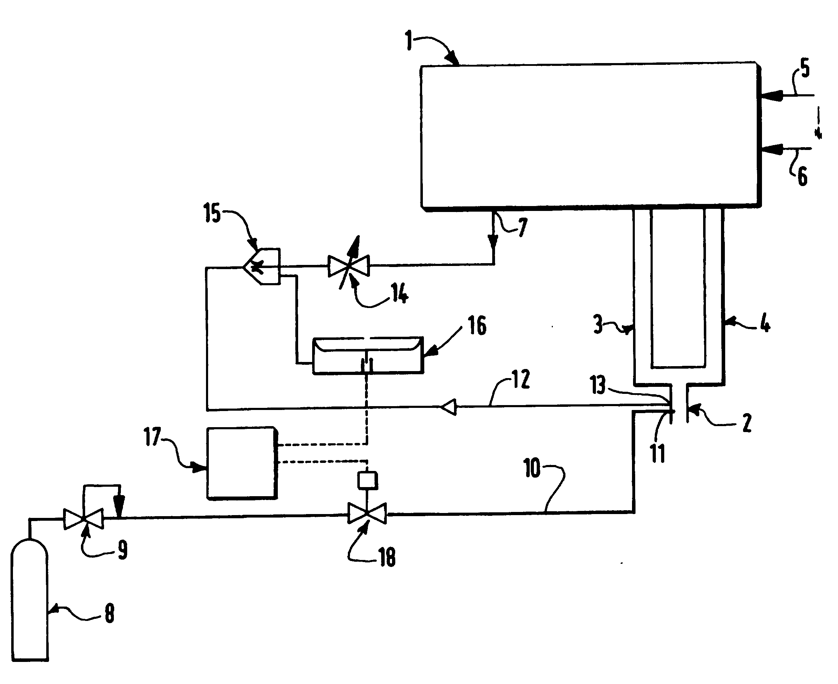

D'autres caractéristiques et avantages de la présente invention ressortiront de la description suivante d'un mode de réalisation, donnée à titre illustratif mais nullement limitatif, faite en relation avec le dessin annexé, sur lequel :
la figure unique représente schématiquement une installation selon l'invention.

Sur la figure unique, on reconnaît un dispositif de fourniture d'un mélange respiratoire ou respirateur 1, tel que décrit dans le document EP-A-347.282 sus-mentionné, couplé à une sonde d'intubation ou un embout d'utilisation 2 par une branche inspiratoire 3 et une branche expiratoire 4 se rejoignant au niveau de l'embout 2. Le respirateur 1 comporte un dispositif de détection de début de phase respiratoire (non représenté), une entrée d'oxygène 5, une entrée d'air 6 et une sortie 7 d'un débit annexe de mélange respiratoire air/oxygène.

Selon l'invention, l'installation comporte une bouteille 8 contenant un gaz inerte, typiquement de l'azote, et une faible quantité de monoxyde d'azote, inférieure à 250 ppm, typiquement d'environ 225 ppm, munie d'un détendeur 9, et reliée, par une ligne 10, à un point 11 dans une partie aval de l'embout ou de sonde 2. La sortie annexe 7 du respirateur 1 est reliée par une ligne 12 à la sonde 2 en un point 13 en amont du débouché 11 de la ligne 10.

La ligne 12 comporte une vanne de réglage 14 et un capteur déprimogène à Venturi 15 couplé à un transducteur de pression 16 fournissant un signal à un bloc de régulation électronique 17 commandant une électrovanne 18 interposée dans la ligne 10, en aval du détendeur 9.

Lors d'une phase inspiratoire du patient, le respirateur 1 fournit à la branche inspiratoire 3 et à la sortie 7 un mélange respiratoire d'air enrichi en oxygène. Le flux de ce mélange respiratoire dans la ligne 12 provoque, dans le capteur 15, une dépression qui est détectée par le transducteur 16 et qui commande l'ouverture de l'électrovanne 18 pour adjoindre, dans le flux de mélange respiratoire fourni par le respirateur 1 à la sonde 2, une quantité dosée de monoxyde d'azote. L'électrovanne 18, du type normalement fermé, est ouverte par le bloc de contrôle 17, en début de phase inspiratoire, pendant une période que l'on peut faire varier par exemple entre 0,1 et 1 seconde, la durée d'ouverture et la pression, fixée par le détendeur 9, étant déterminées de façon à obtenir la concentration en monoxyde d'azote souhaitée en fonction du volume d'inspiration courant de l'utilisateur, typiquement entre 10 et 40 ppm. Le transducteur de pression 16 est avantageusement du type différentiel à membrane, tel que décrit dans le document EP-A-183.593, au nom de la Demanderesse.

De la description qui précède, on comprendra que le circuit d'injection de monoxyde d'azote, qui peut être adjoint à tout type de respirateur préexistant, est totalement indépendant des circuits de gaz respiratoire du respirateur 1, ce qui évite tout risque d'introduction de monoxyde d'azote dans ce dernier, et que son injection s'effectue le plus en aval possible dans le circuit d'inspiration du patient, en minimisant ainsi les risques d'oxydation du monoxyde d'azote par l'oxygène présent majoritairement dans le mélange respiratoire fourni au patient, par le respirateur 1.

Quoique la présente invention ait été décrite en relation avec un mode de réalisation particulier, elle ne s'en trouve pas limitée pour autant mais est au contraire susceptible de modifications et de variantes qui apparaîtront à l'homme de l'art.

## Revendications

1. Installation de fourniture de mélange gazeux aux voies respiratoires d'un utilisateur, comprenant un dispositif (1) de fourniture séquentielle d'un mélange respiratoire dans un embout d'utilisation (2) et comportant un dispositif de détection de début de phase inspiratoire de l'utilisateur, et comportant, en outre, une source de monoxyde d'azote (8) et des moyens (8 ; 18 ; 10), agissant en réponse au dispositif de détection, pour adjoindre (11), dans l'embout d'utilisation (2), des doses controlées de monoxyde d'azote.

2. Installation selon la revendication 1, caractérisée en ce que les moyens d'adjonction comprennent une ligne (10), entre l'embout d'utilisation (2) et la source de monoxyde d'azote (8), comportant un dispositif de régulation (18, 17) sensible à la délivrance d'un flux de mélange respiratoire par le dispositif de fourniture de mélange respiratoire (1).

3. Installation selon la revendication 2, caractérisée en ce que le dispositif de fourniture de mélange respiratoire (1) comporte une sortie annexe (7) de mélange respiratoire et en ce que le dispositif de régulation de doses de monoxyde d'azote (17) comporte des moyens de détection (15, 16) de passage d'un flux de mélange respiratoire par la sortie annexe (7).

4. Installation selon la revendication 3, caractérisée en ce que les moyens de détection comprennent un dispositif déprimogène (15) relié à la sortie annexe (7) et couplé à un capteur de pression (16).

5. Installation selon la revendication 4, caractérisée en ce que le capteur de pression (16) est du type différentiel à membrane.

6. Installation selon l'une des revendications 2 à 5, caractérisée en ce que la source de monoxyde d'azote est constituée d'un réservoir (8) muni d'un détendeur (9).

7. Installation selon la revendication de 6, caractérisée en ce que le réservoir (8) contient un mélange d'azote et de monoxyde d'azote, la teneur en monoxyde d'azote dans le mélange n'excédant pas 250 ppm.

8. Installation selon l'une des revendications précédentes, caractérisée en ce que le dispositif de fourniture de gaz respiratoire (1) est relié à une source d'oxygène (5).

9. Procédé de mise en oeuvre d'une installation selon la revendication 8, caractérisé en ce qu'on règle la teneur en oxygène dans le mélange respiratoire délivré par le dispositif de fourniture (1) à une teneur non inférieure à 21 %, et la teneur en monoxyde d'azote adjoint à une valeur n'excédant pas 50 ppm.

## Patentansprüche

1. Anlage zur Versorgung der Atmungswege eines Benutzers mit einem Gasgemisch, mit einer Vorrichtung (1) zur sequentiellen Lieferung eines Atmungsgemisches in einen Benutzungsstutzen (2) und mit einer Einrichtung zum Erfassen des Beginns der Einatmungsphase des Benutzers und des weiteren mit einer Stickstoffmonoxidquelle (8) und Einrichtungen (8, 18, 10) zur Zugabe (11) kontrollierter Dosen Stickstoffmonoxids in den Benutzungsstutzen (2) in Abhängigkeit von den Erfassungseinrichtungen aufweist.

2. Anlage nach Anspruch 1, dadurch gekennzeichnet, daß die Zugabeeinrichtungen zwischen den Benutzungsstutzen (2) und einer Stickstoffmonoxidquelle (8) eine Leitung (10) mit einer Regelvorrichtung (18, 17) aufweisen, die für die Zufuhr eines Stromes Atmungsgemisch von der Vorrichtung (1) zur Lieferung des Atmungsgemisches empfindlich ist.

3. Anlage nach Anspruch 2, dadurch gekennzeichnet, daß die Vorrichtung (1) zur Lieferung des Atmungsgemisches einen Zusatzausgang (7) für Atmungsgemisch aufweist und daß die Vorrichtung (17) zur Regelung von Dosen Stickstoffmonoxid Einrichtungen (15, 16) zur Erfassung des Durchtritts eines Atmungsgemischstromes durch den Zusatzausgang (7) aufweist.

4. Anlage nach Anspruch 3, dadurch gekennzeichnet, daß die Erfassungseinrichtungen eine Vorrichtung (15) zur Erzeugung eines Unterdrucks aufweisen, die mit dem Zusatzausgang (7) verbunden und an einen Drucksensor (16) angeschlossen ist.

5. Anlage nach Anspruch 4, dadurch gekennzeichnet, daß der Drucksensor (16) mit einer Membran und nach dem Differenzdruckprinzip arbeitet.

6. Anlage nach einem der Ansprüche 2 bis 5, dadurch gekennzeichnet, daß die Stickstoffmonoxidquelle aus einem mit einem Druckminderer (9) versehenen Speicherbehälter (8) besteht.

7. Anlage nach Anspruch 6, dadurch gekennzeichnet, daß der Speicherbehälter (8) ein Gemisch aus Stickstoff und Stickstoffmonoxid enthält, wobei der Stickstoffmonoxidgehalt in dem Gemisch 250 ppm nicht übersteigt.

8. Anlage nach einem der vorhergehenden Ansprüche, dadurch gekennzeichnet, daß die Vorrichtung (1) zur Lieferung des Atmungsgases mit einer Sauerstoffquelle (5) verbunden ist.

9. Verfahren zur Verwendung einer Anlage nach Anspruch 8, dadurch gekennzeichnet, daß man den Sauerstoffgehalt in dem von der Liefervorrichtung (1) gelieferten Atmungsgemisch auf einen Gehalt von nicht weniger als 21 % und den Gehalt an zugegebenem Stickstoffmonoxid auf einen Wert von nicht mehr als 50 ppm regelt.

## Claims

1. Installation for the supply of a gaseous mixture to the respiratory tract of a user, comprising a device (1) for the sequential supply of a respiratory mixture to a user end piece (2) and comprising a device for detecting the start of the user's inhalation phase, and additionally comprising a source of nitrogen monoxide (8) and means (8; 18; 10) acting in response to the detection device in order to add (11), into the user end piece (2), controlled doses of nitrogen monoxide.

2. Installation according to claim 1, characterized in that the addition means comprise a line (10), between the user end piece (2) and the source of nitrogen monoxide (8), comprising a regulation device (18, 17) sensitive to the delivery of a flow of respiratory mixture by the device for the supply of respiratory mixture (1).

3. Installation according to 2, characterized in that the device for the supply of respiratory mixture (1) comprises an auxiliary outlet (7) for respiratory mixture and in that the device for regulating doses of nitrogen monoxide (17) comprises means (15, 16) for detecting the passage of a flow of respiratory mixture via the auxiliary outlet (7).

4. Installation according to 3, characterized in that the detection means comprise a pressure-reducing device (15) connected to the auxiliary outlet (7) and coupled to a pressure sensor (16).

5. Installation according to claim 4, characterized in that the pressure sensor (16) is of the differential type with a diaphragm.

6. Installation according to one of claims 2 to 5, characterized in that the source of nitrogen monoxide is formed by a tank (8) provided with a pressure reducing valve (9).

7. Installation according to claim 6, characterized in that the tank (8) comprises a mixture of nitrogen and nitrogen monoxide, the nitrogen monoxide content of the mixture not exceeding 250 ppm.

8. Installation according to one of the preceding claims, characterized in that the device for the supply of respiratory gas (1) is connected to an oxygen source (5).

9. Method for the application of an installation according to claim 8, characterized in that the oxygen content of the respiratory mixture distributed by the supply device (1) is regulated to a content of no less than 21% and the nitrogen monoxide added is regulated to a content of no more than 50 ppm.
